**Europäisches Patentamt**

**European Patent Office**

**Office européen des brevets**

(11) Numéro de publication: **0 079 283**
**B1**

# FASCICULE DE BREVET EUROPEEN

(45) Date de publication du fascicule du brevet:
**26.02.86**

(21) Numéro de dépôt: **82402040.8**

(22) Date de dépôt: **05.11.82**

(51) Int. Cl.⁴: **G 01 N 1/10** // G21F7/06 .

(54) **Système de mise en circulation continue d'un liquide en vue d'un prélèvement ou d'un contrôle de ce liquide.**

(30) Priorité: **10.11.81 FR 8121023**

(43) Date de publication de la demande:
**18.05.83 Bulletin 83/20**

(45) Mention de la délivrance du brevet:
**26.02.86 Bulletin 86/9**

(84) Etats contractants désignés:
**BE DE GB IT SE**

(56) Documents cités:
**DE - A - 2 053 004**
**FR - A - 2 238 931**
**GB - A - 1 360 346**
**US - A - 2 995 037**
**US - A - 3 834 588**

(73) Titulaire: **COMMISSARIAT A L'ENERGIE ATOMIQUE Etablissement de Caractère Scientifique Technique et Industriel, 31/33, rue de la Fédération, F-75015 Paris (FR)**

(84) Etats contractants désignés: **BE DE GB IT**

(73) Titulaire: **SOCIETE GENERALE POUR LES TECHNIQUES NOUVELLES S.G.N. Société anonyme dite:, 1, rue des Hérons Montigny-le-Bretonneux, F-78184 Saint-Quentin en Yvelines Cedex (FR)**

(84) Etats contractants désignés: **SE**

(72) Inventeur: **Conche, François, 15, rue du Château, F-78130 Les Mureaux (FR)**
Inventeur: **Naujalis, Pierre, 16, Avenue Pierre Curie, F-94310 Orly (FR)**

(74) Mandataire: **Mongrédien, André et al, c/o BREVATOME 25, rue de Ponthieu, F-75008 Paris (FR)**

LIBER, STOCKHOLM 1986

# 0 079 283

## Description

La présente invention a pour objet un dispositif de mise en circulation continue d'un liquide et plus particulièrement de mise en circulation de ce liquide entre une cuve de stockage et un récipient où doit être effectué un prélèvement ou un contrôle.

La principale difficulté qui se présente dans les dispositifs de prélèvement de liquides, notamment en vue d'analyse, est d'obtenir au point de prélèvement un echantillon homogène et représentatif du liquide contenu dans la cuve. Dans la plupart des systèmes utilisés à l'heure actuelle, on fait circuler le liquide entre la cuve de stockage et le point de prélèvement af in d'assurer une bonne homogénéisation et donc une bonne représentativité. Une autre difficulté à résoudre est d' éviter les dépôts qui pourraient se former le long des parois du dispositif, car ceux-ci risquent d'être dissous par un autre liquide lors d'un contrôle ultérieur, faussant ainsi les résultats des mesures. On utilise beaucoup à l'heure actuelle des systèmes dits 'air-lift' dans lesquels de l'air comprimé est insuffle dans une conduite reliant la cuve de stockage au point de prélèvement afin de faciliter la circulation du liquide. Un tel système est décrit, par exemple, dans la brevet US-A-3 834 588. Si un tel système permet une bonne homogénéisation de celui-ci, la présence de bulles d'air dans le liquide peut néanmoins être gênante, notamment dans le cas où le prélèvement se fait à l'aide d'aiguilles creuses sur lesquelles on pique un cruchon dans lequel on a préalablement fait le vide.

La présente invention a justement pour objet un dispositif de mise en circulation continue d'un liquide qui évite les inconvénients mentionnés ci-dessus en permettant non seulement une bonne homogénéisation du liquide et une vidange efficace de l'installation après prélèvement, mais aussi en évitant la présence de bulles d'air dans le liquide au niveau du point de prelèvement.

Selon la principale caractéristique du dispositif objet de l'invention, celui-ci, qui est destine à faire circuler le liquide entre une cuve de stockage et un récipient ou pot de passage dans lequel est effectué le prélèvement ou le contrôle, utilisant une source de gaz comprimé (5) ladit dispositif étant caractéericé en ce qu'il comprend:
- une conduite d'alimentation reliant la cuve a la partie inférieure d'un séparateur situé au-dessus de celle-ci,
- une conduite de communication (3) entre ladite source de gaz comprimé (5) et ladite conduite d'alimentation (4),
- une canalisation d'un séparateur situé au-dessus de séparateur à la partie inférieure du pot de passage, la partie inférieure de ce dernier étant a un niveau plus élevé que celle du séparateur,
- une conduite de retour reliant la partie supérieure du pot de passage à la cuve, et
- une conduite, équipée d'un dévésiculeur, reliant la partie supérieure du séparateur à un appareil permettant de créer une dépression.

De manière classique, l'insuf flation d'air comprimé dans la conduite reliant la cuve au séparateur facilite la montée du liquide. Quant à l'appareil permettant de créer une dépression, qui peut être une source de vide ou un ventilateur, son rôle est de permettre l'élimination, au niveau du séparateur, du gaz présent dans le liquide: c'est donc du liquide parfaitement dégazé qui se rassemble dans la partie basse du séparateur, puis monte progressivement jusqu'au pot de passage.

Selon une première variante de réalisation, la conduite d'alimentation est rectiligne et plonge verticalement dans la cuve de stockage. Dans ce cas, la source de gaz comprimé est en communication avec une conduite débouchant dans la conduite d'alimentation en un point situé à un niveau supérieur à celui de la surface libre du liquide dans la cuve et l'appareil permettant de créer une dépression est une source de vide.

Selon une autre variante de réalisation, la conduite d'alimentation débouche dans la partie inferieure de la cuve et présente dans cette zone une partie recourbée. Dans ce cas, la conduite reliant la source de gaz comprimé à la conduite d'alimentation debouche dans celle-ci en un point situé sous la surface libre du liquide dans la cuve lorsque l'installation est au repos et l'appareil permettant de créer une dépression est un appareil de ventilation.

Enfin, selon une dernière caractéristique du dispositif objet de l'invention, celui-ci comprend une conduite d' évacuation de trop-plein reliant la partie supérieure du séparateur à la conduite de retour.

L'invention apparaitra plus clairement à la lecture de la description qui va suivre, donnée à titre d'exemple purement illustratif et nullement limitatif, en référence aux dessins annexés dans lesquels:
- la figure 1 est une vue schématique représentant une première variante de réalisation d'un dispositif conforme à l'invention, et
- la figure 2 est une vue schématique représentant un deuxième mode de réalisation de ce dispositif.

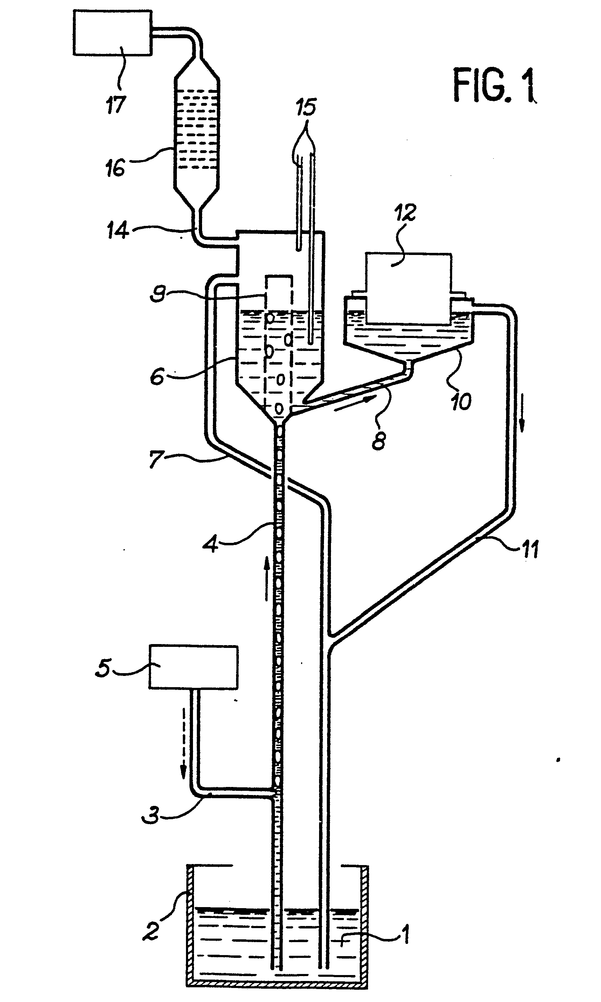

Sur la figure 1, on voit un liquide 1 contenu dans une cuve 2 qui peut être, soit un réservoir de stockage, soit une cuve d'alimentation reliée à un reservoir de volume beaucoup plus important. Une première conduite 4 dite 'conduite d'alimentation'' relie la cuve 2 à la partie inférieure d'un récipient 6 appelé 'séparateur'', situé au-dessus de la cuve 2, et dans lequel est effectuée la séparation du liquide et du gaz comprimé. Un tube perforé, placé à l'intérieur du séparateur 6, joue le rôle de tranquilliseur pour le liquide afin d'éviter une agitation qui pourraît être gênante dans le pot de passage 10, tandis que deux cannes de bullage 15 permettent de contrôler la présence du liquide dans le séparateur et d'en vérifier le niveau. La partie inférieure du séparateur 6 est reliée par une canalisation 8 à la partie inférieure d'un récipient 10 appelé pot de passage dans lequel on effectue le con-trôle de l'état du liquide. On remarquera que la partie inférieure du pot de passage 10 est située à un niveau plus élevé que la partie inférieure du séparateur 6. Le pot de passage 10 est équipé d'un appareil de contrôle 12 qui permet soit de contrôler directement l'état du liquide se trouvant à l'intérieur du pot de passage 10, soit d'effectuer un prélèvement lorsque le contrôle a lieu dans une autre installation. Une conduite de retour 11,

2

branchée à la partie supérieure du pot de passage 10 relie celui-ci à la cuve 2.

Sur la figure, on voit également, branchée à la partie supérieure du séparateur 6, une canalisation 14 équipée d'un dévésiculeur 16 et reliant le séparateur 6 à une source de vide 17. Le dévésiculeur 16 a pour rôle de permettre la séparation des dernières gouttelettes qui auraient pu être entraînées avec le gaz aspiré par la source de vide 17. Une conduite d'évacuation de trop-plein 7 relie la partie supérieure du séparateur 6 à la conduite de retour 11 et donc à la cuve 2. La disposition de l'ensemble est telle que le point de départ de la conduite 7 se trouve à un niveau plus élevé que le point de départ de la conduite de retour 11. On voit enfin sur la figure une conduite d'air comprimé 3 qui met en communication la conduite d'alimentation 4 avec une source d'air comprimé 5. Dans la variante de la figure 1, la conduite d'alimentation 4 est rectiligne et verticale et pénètre dans la cuve 2 à la partie supérieure de celle-ci tandis que la conduite d'air comprimé 3 débouche dans la conduite 4 en un point situé au-dessus de la surface libre du liquide dans la cuve 2.

Le fonctionnement de l'appareil est le suivant: lorsque la source de vide 17 est mise en fonctionnement, il se produit une aspiration dans la conduite d'alimentation 4. Le liquide contenu dans la cuve 2 monte alors le long de cette conduite jusqu'à un point situé au-dessus du point de branchement de la conduite d'air compr imé 3. A ce moment, l'air compr ime est insufflé dans la conduite 4 à travers la conduite 3 et, du fait de la mise sous vide de l'installation, les bulles d'air montent aussitôt dans la conduite 4, entraînant avec elles le liquide contenu dans la cuve. Lorsque celui-ci arrive dans la partie basse du séparateur 6, l'air est évacué à travers la canalisation 14. Comme il se crée ainsi une aspiration continue du liquide de la cuve 2 vers le séparateur 6, le niveau du liquide dans celui-ci, et par conséquent dans la conduite 8, monte progressivement. On comprend aisément que, puisque l'air est éliminé de la masse liquide au niveau du séparateur, c'est du liquide parfaitement degazé qui envahit progressivement, de bas en haut, la conduite 8 puis le pot de passage 10. Lorsque le niveau du liquide dans ce dernier atteint une hauteur suffisante, il redescend dans la cuve 2 à travers la conduite de retour 11. Il s'établit ainsi une circulation continue du liquide, de la cuve 2 au séparateur 6 à travers la conduite 4, puis du séparateur au pot de passage à travers la conduite 8 et enfin du pot de passage à la cuve à travers la conduite de retour 11: cette circulation permet d'homogénéiser le liquide au niveau de l'appareil de contrôle 12. D'autre part, du fait de la présence du séparateur 6 en amont du pot de passage 10, c'est un liquide exempt de gaz qui passe à travers celui-ci améliorant notablement la représentativité des échantillons contrôlés et facilitant le prelèvement. Si, pour une raison quelconque, par exemple un bouchage accidentel de la conduite 11, le niveau du liquide dans le séparateur 6 montait de manière inquiétante, la présence de la conduite d'évacuation de tropplein 7, branchée à la partie supérieure de celui-ci, permet l' évacuation du liquide en direction de la conduite de retour 11 et, de là, à la cuve 2. Lorsque le contrôle ou le prélèvement est terminé, on arrête la source de vide et on ferme l'arrivée d'air comprimé. A ce moment, tout le liquide contenu dans l'installation redescend par gravité dans la cuve 2: en particulier, le liquide contenu dans le pot de passage 10 redescend par la conduite 8 en direction de la partie basse du séparateur 6 et de là dans la cuve 2 à travers la conduite 4. Cette disposition, notamment grâce à l'inclinaison de la conduite 8, assure une vidange immédiate et totale de l'installation et diminue considérablement le risque d'un dépôt qui pourrait fausser les mesures ultérieures.

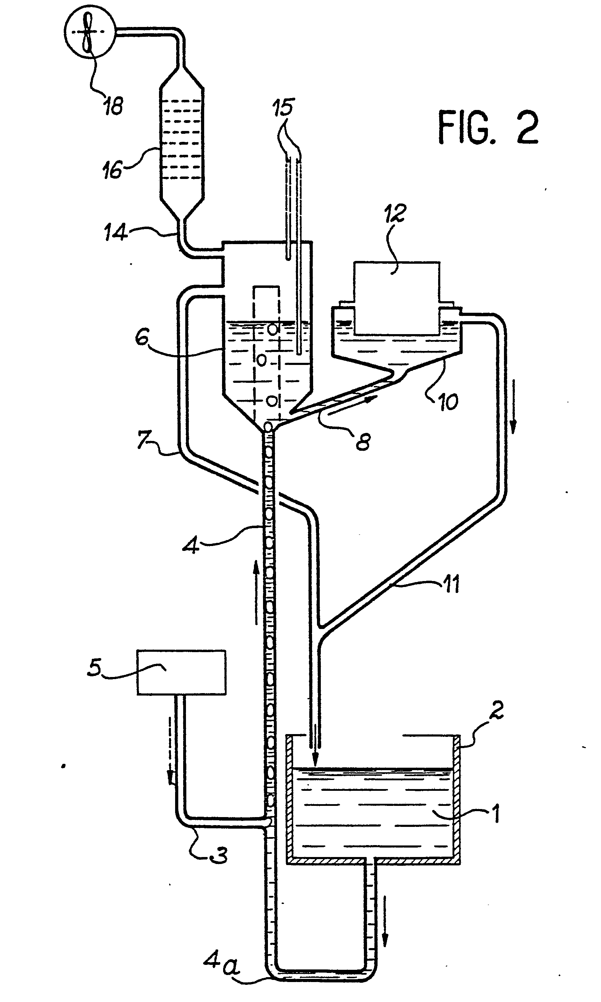

Dans la variante de la figure 2, on voit que la source de vide 17 est remplacée par un appareil de ventilation 18. D'autre part, la conduite d'alimentation 4 ne pénètre plus dans la partie supérieure de la cuve 2, mais débouche dans le fond de celle-ci. Elle présente dans cette zone une partie recourbée 4a qui est constamment remplie de liquide jusqu'à un niveau egal à celui de la surface libre du liquide dans la cuve. La conduite 3 d'alimentation en air comprimé debouche dans la partie basse de la conduite 4 en un point qui se trouve sous la surface libre du liquide lorsque l'installation est au repos. Lorsqu'on envoie l'air comprime à travers la conduite 3, les bulles d'air entrainent immédiatement le liquide le long de la conduite 4. Dans ce cas, la ventilation assurée par l'appareil 18 ne sert qu'à éliminer l'air introduit par la conduite 3 afin de déga zer le liquide qui se rassemble à la partie inférieure du séparateur 6. Le liquide monte donc le long de la conduite d'alimentation 4 jusqu'à la partie basse du séparateur 6 où l'air est éliminé, puis monte le long de la conduite 8 selon le même mécanisme que précédemment. Lors de l'arrêt de l'installation, le liquide redescend dans la conduite 4 jusqu'à un niveau correspondant à celui de la surface libre du liquide dans la cuve 2.

Le dispositif selon l'invention présente de nombreux avantages dont le plus intéressant est d'assurer le passage d'un liquide homogène et parfaitement dégazé au point de prélèvement. D'autre part, lors de l'arrêt de l'installation, il assure une vidange totale et très efficace, ce qui évite le risque d'un dépôt parasite qui pourrait fausser les mesures suivantes.

Si un tel dispositif trouve une application particulièrement intéressante dans les installations de prelèvement d' échantillons liquides radioactifs, notament celles où l'on utilise une aiguille creuse sur laquelle on vient piquer un cruchon dans lequel on a prealablement fait le vide, il peut également s'appliquer à n'importe quelle installation de prélèvement où l'on fait circuler le liquide et où l'on désire que celui-ci soit parfaitement homogène et dégazé.

## Revendications

1. Dispositif de mise en circulation continue d'un liquide entre une cuve de stockage (2) et un recipient ou pot de passage (10) dans lequel est effectué un prélèvement ou un contrôle, utilisant une source de gaz comprimé (5) ladit dispositif étant caractérisé en ce qu' il comprend:

- une conduite d'alimentation (4) reliant la cuve (2) à la partie inférieure d'un séparateur (6) situe auessus de celle-ci,
- une conduite de communication (3) entre ladite source de gaz comprimé (5) et ladite conduite d'alimentation (4),
- une canalisation (8) reliant la partie inférieure dudit séparateur (6) à la partie inférieure du pot de passage (10), la partie inférieure de ce dernier étant à un niveau plus élevé que celle du séparateur (6),
- une conduite de retour (11) reliant la partie supérieure ure du pot de passage (10) à la cuve (2), et
- une conduite (14), équipée d'un dévésiculeur (16), reliant la partie supérieure du séparateur (6) à un appareil (17;18) permettant de créer une dépression.

2. Dispositif selon la revendication 1, caractérisé en ce que l'appareil permettant de créer une dépression est une source de vide (17) et en ce que la source de gaz comprimé (5) est en communication avec une conduite (3) débouchant dans la conduite d'alimentation (4) à un niveau supérieur à celui de la surface libre du liquide (1) dans la cuve (2).

3. Dispositif selon la revendication 1, caractérisé en ce que l'appareil permettant de créer une dépression est un appareil de ventilation (18) et en ce que la conduite de gaz comprimé (3) débouche dans la conduite d'alimentation (4) en un point situé à un niveau inférieur à celui de la surface libre du liquide (1) dans la cuve (2) lorsque l'installation est au repos.

4. Dispositif selon l'une quelconque des revendicati-ons 1 à 3, caractérisé en ce qu' il comprend une conduite d' évacuation de trop-plein (7) reliant la partie supérieure du séparateur (6) à la conduite de retour (11).


**Patentansprüche**

1. Einrichtung zur kontinuierlichen Zirkulation einer Flüssigkeit zwischen einem Vorratsbecken (2) und einem Gefäß oder Durchlaufbehälter (10), in welcher eine Probenahme oder Kontrolle unter Verwendung einer Druckgasquelle (5) ausgeführt wird, wobei die genannte Vorrichtung dadurch <u>gekennzeichnet</u> ist, daß sie
- eine das Becken (2) mit dem unteren Teil eines über diesem angeordneten Separator (6) verbindende Zuleitung (4),
- eine Verbindungsleitung (3) zwischen der Druckgasquelle (5) und der Zuleitung (4),
- eine das untere Teil des Separators (6) mit dem unteren Teil des Durchlaufbehälters (10) verbindende Leitung (6), wobei das untere Teil des Letzteren auf einem höheren Niveau liegt als das des Separators (6),
- eine das obere Teil des Durchlaufbehälters (10) mit dem Becken (2) verbindende Rückleitung (11) und
- eine mit einem Blasenabscheider (16) ausgerüstete Leitung (14) aufweist, welche das obere Teil des Separators (6) mit einer zum Erzeugen eines Unterdrucks ausgebildeten Einrichtung (17, 18) verbindet.

2. Einrichtung nach Anspruch 1, dadurch <u>gekennzeichnet</u>, daß die zum Erzeugen eines Unterdrucks ausgebildete Einrichtung eine Vakuumquelle (17) ist und daß die Druckgasquelle (5) mit der Leitung (3) strömungsverbunden ist, welche auf einem oberhalb des Niveaus der freien Oberfläche der Flüssigkeit (1) im Becken (2) liegenden Niveeu in die Zuleitung (4) einmündet.

3. Einrichtung nach Anspruch 1, dadurch <u>gekennzeichnet</u>, daß die zum Erzeugen eines Unterdrucks ausgebildete Einrichtung eine Lüftervorrichtung (18) ist und daß die Druckgasleitung (3) an einer Stelle in die Zuleitung (4) mündet, welche im Ruhezustand der Anlage auf einem unterhalb des Niveaus der freien Oberfläche der Flüssigkeit (1) im Becken (2) liegenden Niveau liegt.

4. Einrichtung nach einem der Ansprüche 1 bis 3, dadurch <u>gekennzeichnet</u>, daß sie eine überlaufleitung (7) aufweist, welche das obere Teil des Separators (6) mit der Rückleitung (11) verbindet.


**Claims:**

1. Apparatus for continuous circulation of a liquid between a storage vessel (2) and a receiver or a holding tank (10) in which sampling or checking is conducted, employing a source of compressed gas (5), said apparatus being characterised in that it comprises:
a supply conduit (4) connecting the vessel (2) to the lower part of a separator (6) located above it,
a communication conduit (3) between said source of compressed gas (5) and said supply conduit (4),
a pipe (8) connecting the lower part of said separator (6) to the lower part of the holding tank (10), the lower part of the latter having a level above that of the separator (6),
a return conduit (11) connecting the upper part of the holding tank (10) to the vessel (2), and
a conduit (14) equipped with a bubble-separator (16) connecting the upper part of the separator (6) to a pressure-reduction apparatus (17, 18).

2. Apparatus according to claim 1, characterised in that the pressure-reduction apparatus is a vacuum source (17) and in that the source of compressed gas (5) is in communication with the conduit (3) opening into the supply conduit (4) at a level above that of the free surface of liquid (1) in the vessel (2).

3. Apparatus according to claim 1, characterised in that the pressure-reduction apparatus is a ventilation apparatus (18) and in thät the compressed gas conduit (3) opens into the supply conduit (4) at a point located at a level below that of the free surface of liquid (1) in the vessel (2) when the installation is in its resting state.

4. Apparatus according to any one of claims 1 to 3, characterised in that it comprises an overflow conduit (7), connecting the upper part of the separator (6) to the return conduit (11).

FIG. 1

FIG. 2